# EUROPEAN PATENT APPLICATION

(11) **EP 4 728 879 A1**
(43) Date of publication of application: **22.04.2026**
(21) Application number: 24831994.9
(22) Date of filing: 26.06.2024
(51) Int. Cl.: A23L 33/10, A61K 31/352, A61K 31/7048, A61K 45/00, A61P 25/28, A61P 43/00, C12N 9/99

(54) **MEMORY DISORDER AMELIORATING AGENT**

(30) Priority: 28.06.2023 JP 2023105921
(71) Applicant: National University Corporation Kobe University, Kobe-shi, Hyogo 657-8501 (JP)
(72) Inventor: SHIRAI, Yasuhito, Kobe-shi, Hyogo 657-8501 (JP)
(74) Representative: Müller-Boré & Partner Patentanwälte PartG mbB
(86) International application number: PCT/JP2024/023169
(87) International publication number: WO 2025/005135

(57) **Abstract**

The present invention provides a novel memory disorder ameliorating agent. A PKCγ activity inhibitor is useful as an active ingredient for a memory disorder ameliorating agent.

## Description

### Technical Field

The present invention relates to a memory disorder ameliorating agent.

### Background Art

Memory disorder is known to occur due to irreversible neurodegeneration such as Alzheimer dementia. Memory disorder caused by irreversible neurodegeneration as a factor is basically not ameliorated because the factor is irreversible, and as a countermeasure, it only slows the progression.

On the other hand, it is known that memory disorder occurs due to another factor. Examples thereof include depression, trauma, stress, brain fog, and the like.

For example, NPL 1 describes that there is memory disorder as a neuropsychological symptom of depression, and a brain function of depression is recovered in accordance with clinical recovery. In addition, there is memory disorder as a symptom of brain fog that is rapidly increasing due to the sequelae of novel coronavirus infection. NPL 2 describes that brain fog often gradually recovers over time, and NPL 3 describes that in more than half of the sequelae, amelioration in symptoms is likely to be felt within 5 months although there are individual differences. Further, NPL 4 describes that a main symptom of dissociative amnesia caused by a psychological trauma or stress is memory disorder that is not consistent with normal forgetting, and that most patients recover lost memory and the amnesia is eliminated. That is, the memory disorder caused by these other factors is fundamentally different from the memory disorder caused by irreversible neurodegeneration described above in that the memory disorder is a symptom in which recovery is expected.

### Citation List

### Non Patent Literature

NPL 1: Yasumasa Okamoto, Functional Brain Basis of Pathophysiology in Depression, Psychiatria et Neurologia Japonica, Vol. 111, No. 11 (2009), p. 1330-1344
NPL 2: Kumiko Arai, What is brain fog that is rapidly increasing due to sequelae of novel coronavirus? Difference from dementia and forgetfulness, [online], 2023/2/13, [searched on April 12, 2023], Internet <URL: https://nishiharu-clinic.com/2023/02/13/korona-bureinfogu/>
NPL 3: Hidehaya Hatsumura, What is "brain fog" of corona sequelae?, [online], 2022/10/13, [searched on April 12, 2023], Internet <URL: https://utuyobo.com/column/brain-fog/>
NPL 4: David Spiegel, MSD Manual Professional Version 08. PSYCHIATRIC DISORDERS/DISSOCIATIVE DISORDERS/DISSOCIATIVE AMNESIA, [online], March 2021, Merck & Co., Inc., Rahway, NJ, USA, [searched on April 12, 2023], Internet <URL: https://www.msdmanuals.com/jajp/%E3%83%97%E3%83%AD%E3%83%95%E3%82%A7%E3%83%83%E3%82%B 7%E3%83%A7%E3%83%8A%E3%83%AB/08-%E7%B2%BE%E7%A5%9E%E9%9 A%9C%E5%AE%B3/%E8%A7%A3%E9%9B%A2%E7%97%87%E7%BE%A4/%E8 %A7%A3%E9%9B%A2%E6%80%A7%E5%81%A5%E5%BF%98>

### Summary of Invention

### Technical Problem

In order to ameliorate memory disorder, it is common to perform treatment for removing an onset factor. That is, in order to ameliorate memory disorder caused by depression, trauma, or stress, it is necessary to treat the factor, and drugs of the type used for these treatments generally need to be applied carefully in consideration of the risk of side effects. In addition, regarding the brain fog of the sequelae of the novel coronavirus infection, the therapy has not been clarified since the clear cause is not known. Under such a background, it is desirable to provide a new component that responds to memory disorder based on a clear mechanism of action.

Therefore, an object of the present invention is to provide a memory disorder ameliorating agent with a clear mechanism of action.

### Solution to Problem

The present inventors have found that, using a DGKβ knockout mouse that has been found to exhibit enhanced PKCγ phosphorylation (abnormal PKCγ activity) in the brain as a unique memory disorder model, the memory disorder is ameliorated together with the normalization of PKCγ activity in the brain by allowing the DGKβ knockout mouse to take a PKCγ activity inhibitor. The present invention has been accomplished by further studies based on this knowledge.

That is, the present invention provides inventions of the following aspects.

Item 1. A memory disorder ameliorating agent containing a PKCγ activity inhibitor as an active ingredient.

Item 2. The memory disorder ameliorating agent according to item 1, in which the PKCγ activity inhibitor is a flavonoid selected from the group consisting of scutellarin, baicalin, baicalein, luteonin, apigenin, apigenin glucoside, and salts thereof.

Item 3. The memory disorder ameliorating agent according to item 1 or 2, which is used for ameliorating memory disorder not caused by accumulation of amyloid β.

Item 4. The memory disorder ameliorating agent according to any one of items 1 to 3, which is used for ameliorating memory disorder not caused by exposure to general anesthesia.

Item 5. The memory disorder ameliorating agent according to any one of items 1 to 4, which is used for ameliorating memory disorder not caused by cerebral infarction.

Item 6. A food containing the memory disorder ameliorating agent according to any one of items 1 to 5.

Item 7. Use of a PKCγ activity inhibitor for the production of a memory disorder ameliorating agent.

Item 8. A PKCγ activity inhibitor used in a memory disorder ameliorating agent.

Item 9. A method for ameliorating memory disorder, including a step of administering an effective amount of a PKCγ activity inhibitor to a subject in need of amelioration of memory disorder.

### Advantageous Effects of Invention

According to the present invention, a new memory disorder ameliorating agent with a clear mechanism of action is provided.

### Brief Description of Drawings

[Fig. 1] Fig. 1 shows the results of confirming the effect of ameliorating memory disorder in mice orally administered with a PKCγ activity inhibitor by Y-maze test.
[Fig. 2] Fig. 2 shows the results of confirming the activity of PKCs in the cerebral cortex of mice administered with a PKCγ activity inhibitor by Western blotting.
[Fig. 3] Fig. 3 shows the results of confirming the activity of PKCs in the hippocampus of mice administered with a PKCγ activity inhibitor by Western blotting.
[Fig. 4] Fig. 4 shows the results of confirming the PKCγ activity inhibitory action of various flavonoids.

### Description of Embodiments

### 1. Memory disorder ameliorating agent

The memory disorder ameliorating agent of the present invention contains a PKCγ activity inhibitor as an active ingredient.

### 1-1. Active ingredient

The PKCγ activity inhibitor is not particularly limited as long as it is a compound having an action of inhibiting phosphorylation of PKCγ. Preferable examples of the PKCγ activity inhibitor used in the present invention include flavonoids such as scutellarin (the following formula (1)), baicalin (the following formula (2)), baicalein (the following formula (3)), luteonin (the following formula (4)), apigenin (the following formula (5)), apigenin glucoside (the following formula (6)), and salts thereof.

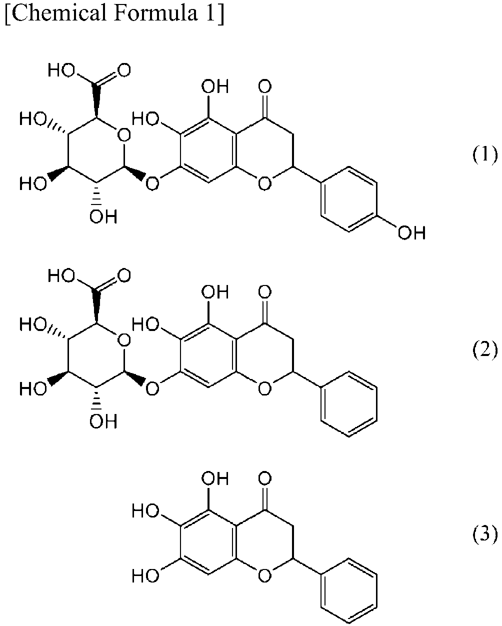

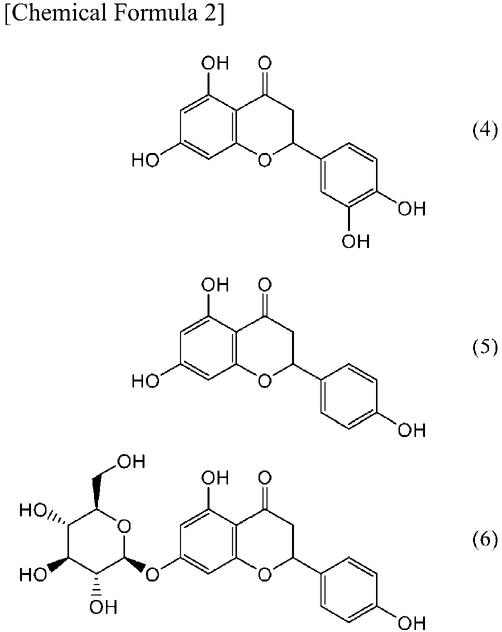

In addition, the salt is not particularly limited as long as it is a pharmaceutically or food acceptable salt, and examples thereof include alkali metal salts such as sodium salts, and the like.

In the memory disorder ameliorating agent of the present invention, among these PKCγ activity inhibitors, one type may be contained alone, or a plurality of types may be contained in combination. Among these PKCγ activity inhibitors, scutellarin, baicalin, luteolin, and apigenin are preferred, baicalin and luteolin are more preferred, and baicalin is further preferred.

The blending amount of the PKCγ activity inhibitor contained in the memory disorder ameliorating agent of the present invention is not particularly limited, and can be appropriately set according to the preparation form and/or specific use, and the like of the memory disorder ameliorating agent, and is, for example, 40 to 100 wt%, preferably 60 to 90 wt%.

### 1-2. Other components

The memory disorder ameliorating agent of the present invention may or may not further contain additives and/or bases according to the preparation form and use as long as the effects of the present invention are not impaired, in addition to the above active ingredients. Such additives and bases that may or may not be contained are not particularly limited as long as they are pharmaceutically acceptable, and examples thereof include excipients, binders, disintegrating agents, lubricants, isotonizing agents, plasticizers, dispersants, emulsifiers, solubilizing agents, wetting agents, stabilizers, suspending agents, pressure sensitive adhesives, gelling agents, coating agents, gloss agents, water, fats and oils, waxes, hydrocarbons, fatty acids, higher alcohols, esters, water-soluble polymers, surfactants, metal soaps, lower alcohols, polyhydric alcohols, pH adjusters, buffering agents, antioxidants, ultraviolet inhibitors, preservatives, flavoring agents, fragrances, powders, thickeners, colorants, chelating agents, sweeteners, and the like. These additives may be used alone or in combination of two or more types. Also, the contents of these additives and bases are appropriately set according to the types of additives and bases to be used, the preparation form and use of the memory disorder ameliorating agent of the present invention, and the like.

Moreover, the memory disorder ameliorating agent of the present invention may or may not contain other nutritional components and/or pharmacological components as long as the effects of the present invention are not impaired, in addition to the above active ingredients. Such nutritional component and pharmacological component are not particularly limited as long as they are pharmaceutically acceptable, and examples thereof include antacids, stomachics, digestive agents, antiflatulents, antispasmodics, mucosal repair agents, anti-inflammatory agents, astringents, antiemetics, antitussives, expectorants, anti-inflammatory enzymes, sedative-hypnotics, antihistamines, caffeines, cardiotonic diuretics, antibacterial agents, vasoconstrictors, vasodilators, local anesthetics, herbal extracts, vitamins, menthols, and the like. These nutritional components and/or pharmacological components may be used alone or in combination of two or more types. In addition, the content of these components is appropriately set according to the type of components to be used.

### 1-3. Preparation form

The form and property of the memory disorder ameliorating agent of the present invention are not particularly limited as long as it contains the above active ingredients.

The administration form of the memory disorder ameliorating agent of the present invention includes both an oral administration form and a parenteral administration form. Therefore, the memory disorder ameliorating agent of the present invention can be prepared as an oral preparation, an injection, a drip, a nasal drop, or the like. Among them, the memory disorder ameliorating agent of the present invention is preferably an oral agent that can be easily administered (ingested) on a daily and/or continuous basis.

Also, the property of the memory disorder ameliorating agent of the present invention may be liquid or solid. Examples of the liquid agent include liquid agents, beverage preparations, emulsions, suspensions, spirits, syrups, elixirs, including soft extracts, and the like), and examples of the solid agent include tablets, pills, powders, fine granules, granules, tablets, capsules (including hard capsules and soft capsules), troches, chewables, dry extracts, and the like. When the memory disorder ameliorating agent of the present invention is solid, it may be in a sustained or sustained release dosage form, or may be mixed with water or the like at the time of administration or ingestion.

The memory disorder ameliorating agent of the present invention can also be used as an additive or the like that adds an additional value to foods, pharmaceuticals, quasi-drugs, and the like. Examples of foods to which the memory disorder ameliorating agent of the present invention is blended include general foods and beverages and health functional foods (including foods for specified health use, nutritive functional foods, foods with function claims, and the like). Among them, the memory disorder ameliorating agent of the present invention contains flavonoids which can be ingested as a food component as an active ingredient, and therefore is preferably blended in a health functional food which can be easily ingested daily and/or continuously. In addition, the memory disorder ameliorating agent of the present invention exhibits an effect based on the action of reducing PKCγ phosphorylation (normalizing PKCγ activity), and therefore is particularly preferably blended in a food for specified health use or a food with function claims.

### 1-4. Production method

The method for producing a memory disorder ameliorating agent of the present invention may be carried out according to a conventionally known normal formulation procedure according to various forms, properties, and intended use using the above active ingredients and other ingredients blended as necessary.

### 1-5. Use

The memory disorder ameliorating agent of the present invention is used for the purpose of ameliorating memory disorder through the normalization of PKCγ activity. The memory disorder is not particularly limited as long as it is caused by inhibition of PKCγ activity. Examples thereof include memory disorder caused by aging, depression, trauma, stress, and/or brain fog. In the memory disorder ameliorating agent of the present invention, the memory disorder is preferably a memory disorder not caused by either irreversible neurodegenerative disease or accumulation of amyloid β, a memory disorder not caused by general anesthesia (in particular, isoflurane) exposure, and a memory disorder not caused by cerebral infarction.

Also, in the memory disorder, a target of memory is not particularly limited, but spatial memory (that is, memory required in an action such as searching for a target, heading to a destination, or returning to an original place) is preferred.

### 1-6. Dose

The dose of the memory disorder ameliorating agent of the present invention is, for example, 0.1 g/day/60 kg or more, preferably 0.3 g/day/60 kg or more, more preferably 0.5 g/day/60 kg or more, and still more preferably 1 g/day/60 kg or more in terms of PKCγ activity inhibitor in a dose to humans.

The administration (ingestion) method of the memory disorder ameliorating agent of the present invention is not particularly limited, and for example, the administration (ingestion) can be performed once or multiple times a day orally or parenterally, and preferably once or 2 to 3 times a day orally.

### 2. PKCγ activity inhibitor

Among the isoflavones used as the PKCγ activity inhibitor as the active ingredient in the above memory disorder ameliorating agent of the present invention, the PKCγ activity inhibitory action is not known for baicalin (the above formula (2)), baicalein (the above formula (3)), luteonin (the above formula (4)), apigenin (the above formula (5)), apigenin glucoside (the above formula (6)), and salts thereof. Therefore, the present invention further also provides a PKCγ activity inhibitor containing, as an active ingredient, a compound selected from the group consisting of baicalin, baicalein, luteonin, apigenin, apigenin glucoside, and salts thereof. These PKCγ activity inhibitors can be used for amelioration of any symptom caused by abnormal PKCγ activity.

### Examples

Hereinafter, the present invention will be more specifically described with reference to Examples, but the present invention is not limited thereto.

### [Test Example 1]

### (Memory disorder model mice)

It has been found by the present inventor that DGKβ, which is one subtype among diacylglycerol kinase (DGK) of which 10 subtypes have been found in mammals, is abundant particularly in the hippocampus, cerebral cortex, and striatum of the brain, and rapidly increased in a period in which a neural network is developed, and also is expressed in a neuron-specific manner and promotes neurite outgrowth. Mice in which DGKβ was knocked out (DGKβKO mice) were prepared as a memory disorder model. The memory disorder model mouse is not a mouse showing accumulation of Aβ in the brain, is not an irreversible neurodegenerative disease model mouse, is not a mouse exposed to general anesthesia such as isoflurane, or is not a cerebral infarction model mouse.

Scutellarin, a PKCγ activity inhibitor, was orally administered to the memory disorder model mice at a dose of 100 mg/mouse for 10 days. Separately, normal mice were prepared, and scutellarin, the PKCγ activity inhibitor, was similarly orally administered to the normal mice at a dose of 100 mg/mouse for 10 days.

### (Memory disorder evaluation system)

A Y-maze device (Y-maze) was used. The Y-maze is a device in which three arms of the same size are radially connected at intervals of 120°, and is used for a spontaneous alternation behavior test, which is one of spatial memory evaluation tests. As an evaluation index of spatial memory, a value obtained by dividing the number of times of entry to different arms continuously three times by a value obtained by subtracting 1 from the total number of entries to the arm and then multiplying the obtained value by 100 (Cross-reaction, Alternations) was used. A lower Alternations indicates a higher degree of memory disorder.

### (Results)

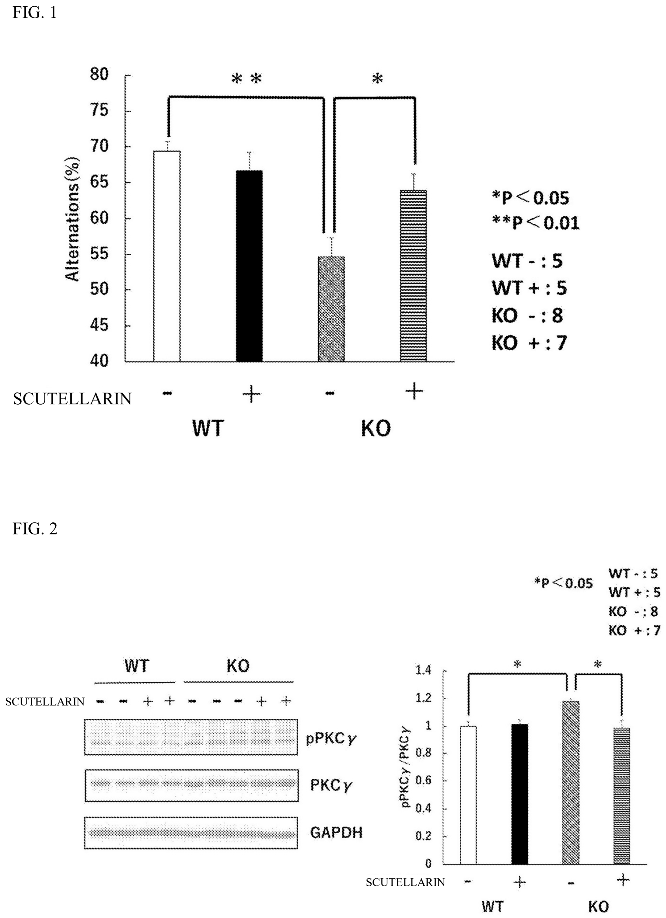

The results are shown in Fig. 1. In the figure, WT represents the results of the normal mice, KO represents the results of the memory disorder model mice, - represents the results without scutellarin administration, and + represents the results with scutellarin administration. In addition, a numerical value described in a format such as "WT -: 5" represents the number of mice (the same applies to the following figures). As shown in Fig. 1, in the memory disorder mouse (KO) group without administration of scutellarin (-), the value of Alternations was significantly low, and it was revealed that memory disorder occurred. Furthermore, it was observed that by administering scutellarin (+), the value of Alternations was significantly high, and the memory disorder in the memory disorder mouse (KO) group was improved to a level close to that of the normal mouse (WT).

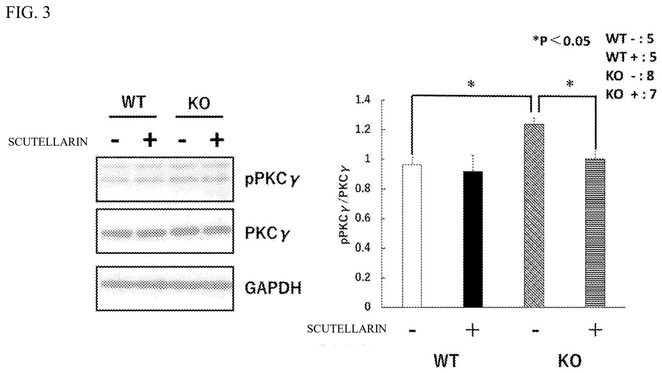

The cerebral cortex and hippocampus of mice administered with scutellarin were subjected to Western blotting, and activation of PKC was evaluated. The results are shown in Fig. 2 (cerebral cortex) and Fig. 3 (hippocampus).

As shown in Figs. 2 and 3, it was observed that abnormal activity of PKCγ in the cerebral cortex and hippocampus was observed in the memory disorder model mouse (KO) group without scutellarin administration (-), whereas it was recognized that the activity of PKCγ was improved to a normal level in the memory disorder model mouse (KO) group with scutellarin administration (+).

### [Test Example 2]

The PKCγ activity inhibitory action of various flavonoids was tested. Scutellarin (Scu), baicalin (Bai), luteolin (Lut), and apigenin (Api) were used as various flavonoids. A plasmid encoding GFP-PKCγ was introduced into COS7 cells by electroporation and cultured for 48 hours to express GFP-PKCγ. After treating the cells with TPA (12-O-Tetradecanoylphorbol-13-acetate, activator of PKCγ) and each flavonoid, the cells were ground and proteins were subjected to SDS-PAGE. Thereafter, Western blotting was performed for PKCγ and phosphorylated PKCγ (activated form).

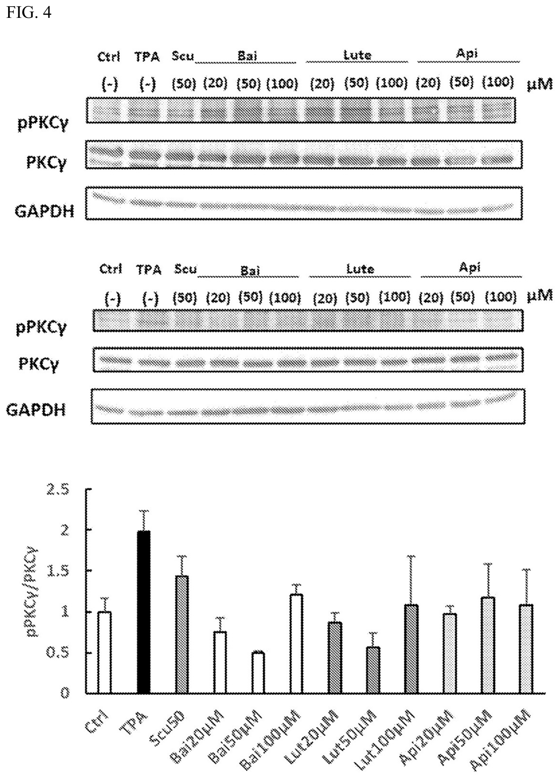

The obtained electrophoresis results (n=2) are shown in Fig. 4. As shown in Fig. 4, the PKCγ activity inhibitory action was observed not only for scutellarin (Scu) but also for baicalin (Bai), luteolin (Lut), and apigenin (Api). From this result, even when baicalin (Bai), luteolin (Lut), and apigenin (Api) are administered to the memory disorder model mice of Test Example 1, a similar memory disorder ameliorating effect can be reasonably inferred.

## Claims

1. A memory disorder ameliorating agent comprising a PKCγ activity inhibitor as an active ingredient.

2. The memory disorder ameliorating agent according to claim 1,
wherein the PKCγ activity inhibitor is a flavonoid selected from the group consisting of scutellarin, baicalin, baicalein, luteonin, apigenin, apigenin glucoside, and salts thereof.

3. The memory disorder ameliorating agent according to claim 1, which is used for ameliorating memory disorder not caused by accumulation of amyloid β.

4. The memory disorder ameliorating agent according to claim 1, which is used for ameliorating memory disorder not caused by exposure to general anesthesia.

5. The memory disorder ameliorating agent according to claim 1, which is used for ameliorating memory disorder not caused by cerebral infarction.

6. A food comprising the memory disorder ameliorating agent according to claim 1.
